# EUROPEAN PATENT APPLICATION

(11) **EP 2 913 065 A1**
(43) Date of publication of application: **02.09.2015**
(21) Application number: 13849310.1
(22) Date of filing: 27.09.2013
(51) Int. Cl.: A61K 49/04, A61K 49/08, A61K 9/16, A61P 35/00

(54) **ULTRASOUND CONTRAST MEDIUM IN WHICH NANOPARTICLES CONTAINING DRUG ARE COMBINED, AND PREPARATION METHOD THEREFOR**

(30) Priority: 25.10.2012 KR 20120119276; 19.06.2013 KR 20130070037
(71) Applicant: Sogang University Research Foundation, Seoul 121-742 (KR)
(72) Inventor: KIM, Hyuncheol, Seoul 150-010 (KR); MOON, Hyungwon, Seoul 157-922 (KR); SONG, Tai-Kyong, Seoul 110-846 (KR); CHANG, jin Ho, Seoul 158-773 (KR); YOO, Yang Mo, Goyang-si Gyeonggi-do 411-730 (KR)
(74) Representative: Fiesser, Gerold Michael
(86) International application number: PCT/KR2013/008647
(87) International publication number: WO 2014/065513

(57) **Abstract**

The present invention relates to an ultrasound contrast agent with drug-encapsulating nanoparticles and a method for preparing the same that increase drug delivery capabilities and drug penetration effects. According to the present investion, exposure to the foucused ultrasound triggers the cavitation of an ultrasound contrast agent, resulting in the local release of drug-encapsulating nanoparticles, which are conjugated onto the surface of the ultrasound contrast agent, and enhaced penetration of nanoparticles and drug delivery capbilities. Moreover, the present invention simultaneously and selectively diagnoses and treats cancers by conjugation cancer-targeting ligands on the surface of nanoparticles.

## Description

### Technical Field

The present invention relates to an ultrasound contrast agent with nanoparticles encapsulting drugs and a method for preparing the same and, more specifically, to an ultrasound contrast agent with nanoparticles and a method for preparing the same that enhance drug transport capabilities and drug penetration effects.

### Background Art

Ultrasound imaging systems have an advantage in that they diagnose disease symptoms in real time, promptly produce the results, are more simple in configuration than MRIs and CTs and cost less. When administrating a contrast agent then emitting ultrasound waves, the ultrasound waves are to reflect from microbubbles included in the agent making the image of an internal organ more obvious. Such an ultrasound contrast agent has evolved since Gramiak and Shah found that ultrasound signals were augmented after injecting microbubbles in blood vessels. Contrast agents known thus far include small air bubbles enclosed by gelatin shell as disclosed in US Patent No. 4,276,885; small gaseous bodies having solid peripheral wall as disclosed in US Patent No. 4,265,251; small air bubbles using particulates of solid crystalline compounds such as galactose as disclosed in the European Patent Gazette No. 52575; small air bubbles using fatty acids as disclosed in European Patent Publication No. 0122624; and small air bubbles fabricated using fatty acids and surfactants as disclosed in Korean Patent No. 1989-2989. Commercial contrast agents that take effects in arterial blood include Levovist (Shering), and Albunex and Optison (Mallinckrodt, USA), etc.

Meanwhile, as medical and therapeutic technologies develop, theragnosis is vigorously studied to simultaneously execute diagnoses and treatments currently provided. Ultrasound contrast agents are also attempted in this regard to simultaneously execute diagnoses and treatment by delivering drugs in an ultrasound contrast agent as a carrier. On the downside, ultrasound contrast agents typically employed are rather restricted in that the capacity to contain drugs is very small, which otherwise may dramatically increase treatment efficacy.

### Disclosure of Invention

### Technical Problem

The present invention provides an ultrasound contrast agent that simultaneously executes diagnosis and therapy by increasing drug transport capabilities and drug penetration effects by exposure of focused ultrasound.

To achieve the objectives, the present invention provides a functional ultrasound contrast agent that maximizes drug penetration capabilities and sustainedly elutes the drug into cells.

### Technical Solution

An ultrasound contrast agent according to an aspect of the present invention comprises microbubbles, which are internally filled with gas, and nanoparticles, which contain drug and are bonded onto the surfaces of the microbubbles.

Another aspect of the present invention relates to a method for preparing an ultrasound contrast agent including a phase that fabricates microbubbles and nanoparticles that contains drugs, respectively; and a phase that mixes the nanoparticles and microbubbles at a predetermined ratio so that the nanoparticles are bound on to the surfaces of the microbubbles.

### Advantageous Effects of Invention

An ultrasound contrast agent according to the present invention increases drug transport capabilities due to nanoparticles, which are bound on to the surface of the ultrasound contrast agent and contain drugs. Moreover, the present invention simultaneously and selectively diagnoses and treats cancers by fabricating nanoparticles utilizing cancer-targeting proteins and maximizes therapeutic efficacy by promoting drug penetrating effects into cells.

### Description of Drawings

FIG 1 is a conceptual diagram of an ultrasound contrast agent according to an embodiment of the present invention.
FIG 2 illustrates a fabrication process of an ultrasound contrast agent according to the present invention.
FIG 3 illustrates confocal fluorescence microscopic images of microbubbles, HSA-NPs(human serum albumin nanoparticles) that contain drugs, and an ultrasound contrast agent that combines the microbubbles and HSA-NPs, respectively, according to the present invention.
FIG 4 illustrates size distribution of an ultrasound contrast agent combined with HSA-NPs according to the present invention.
FIG 5 illustrates an ultrasound image of Embodiment 1 of the present invention and water as a control, both in an agarose phantom, taken by a commercial ultrasound diagnosis device in harmonic mode.
FIG 6 illustrates the harmonic elements selectively collected among the ultrasound waves, which reflect from or transmit an ultrasound contrast agent according to the present invention, obtained by a hydrophone.
FIG 7 illustrates peaks the HSA-NPs-combined microbubbles of Embodiment 1 (left) and water (right), respectively, emit after being emitted by ultrasound waves at 3 MHz.
FIG 8 illustrates long-term stability, against ultrasound waves, of an ultrasound contrast agent of Comparative Example 1 that provides HSA-NPs-unbonded-microbubbles and Embodiment 1, respectively, according to the present invention.
FIG 9 illustrates the test results that verified, using HSA-NPs that contain PTX(paclitaxel), antitumor effects at the cellular level
FIG 10 illustrates the test results that verified the ultrasound wave imaging effects, using an animal model employing MCF-7-transplanted Balb/C nude mice, of Embodiment 1 and Comparative Example 1, respectively.
FIG 11 is a Matlab plot that illustrates the intensity for quantitative comparison, in a tumor, of the imaging effects illustrated in FIG 10.
FIG 12 illustrates the survival ratio of the animal model employing MCF-7-transplanted Balb/C nude mice the results of which were obtained at the time the model showed a symptom of 0.5 mm.

### Mode of Invention

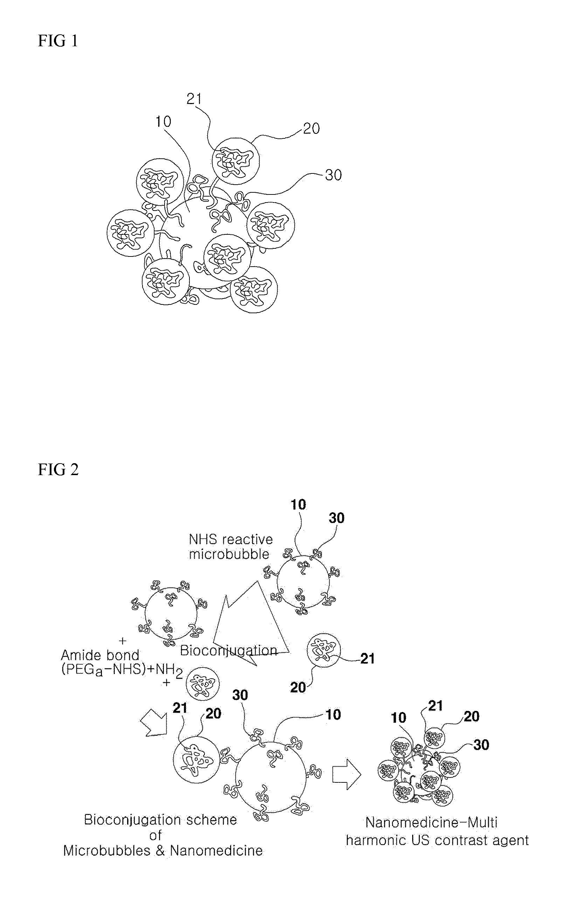

The present invention may be entirely achieved based on the description of the present specification. The description undermentioned must be interpreted to state preferred and specific examples of the present invention whereas the present invention is not limited thereto. FIG 1 is a conceptual diagram of an ultrasound contrast agent according to an embodiment of the present invention. As illustrated in FIG 1, an ultrasound contrast agent according to the present invention includes microbubbles 10 and nanoparticles 20.

The microbubbles 10 may be microspheres filled with gas, preferably liposomes filled with gas or gas-forming emulsions.

The liposomes are formed by amphiphilic compounds that include phospholipids. Such amphiphilic compounds are arrayed on the interface between a medium that is typically aqueous and an organic solvent that is basically water-insoluble, and stabilize the solvent microdroplets thus emulsified. The amphiphilic compounds include a compound having molecules comprising a hydrophilic head, for example polar or ionic radical, that can react to aqueous media and a hydrophobic tail, for example hydrocarbon chain, that can react to, for example, an organic solvent. The amphiphilic compound refers to a compound that stabilize a mixture that is otherwise typically immiscible such as mixture of two kinds of liquid, for example water and oil, immiscible to each other, mixture of liquid and gas, for example air microbubbles in water, or mixture of liquid and insoluble particulates, for example metallic nanoparticles in water. Particularly, the present invention provides, as an amphiphilic compound, liposomes internally filled with inert gas by injecting the inert gas and water in thin phospholipid film then by ultrasound processing the phospholipid thin film.

An amphiphilic phospholipid compound contains at least one phosphate group and at least one, preferably two, lipophilic long-chain hydrocarbon radicals.

The amphiphilic compound may be selected from known such compounds including 1,2-distearoyl-sn-glycero-3-phosphocholine(DSPC), 1,2-diacyl-sn-glycero-3-phosphoethanolamine(DOPE), 1,2-Dimyristoyl-snglycero-3-phosphatidylethanolamine(DMPE), 1,2- distearoyl-sn-glycero-3-phosphoethanolamine(DSPE), etc.

Transformed phospholipid compounds may also be used as the amphiphlic compound. Examples of transformed phospholipid compounds that contain PEG(polyethylene glycol) include DMPE-PEG(phosphatidylethanolamine-polyethylene glycol), DSPE-PEG(phosphoethanolamine -polyethylene glycol), etc.

An amphiphilic phospholipid compound provided for the present invention may include, preferably, NHS(N-hydroxysuccinimide) that forms amide bonds.

The present invention may additionally include amphiphilic materials, besides the amphiphilic compounds, such as lysolipid, stearic acid, polyethyleneglycol, polyoxyethylene fatty acid ester, polyoxyethylene fatty acid stearate, polyoxyethylene fatty alcohol, etc.

The gas to be internally filled in the liposome may be unrestrictedly selected from known gases such as carbon dioxide, helium, nitrogen, argon, sulfur hexafluoride, perfluorinated gases, etc. and may be preferably fluoride that contains fluorine gas such as C3F8(perfluoropropane), SF6(sulfur hexafluoride), perfluoropentane, decafluorobutane, perfluorohexane, etc.

The microbubbles may have a diameter of 0.1 to 10 µm or, preferably a diameter that ranges from 1 to 10 µm.

The nanoparticles 20 internally contains drugs 21 and may form self-aggregates such as albumin.

Proteins that aggregate are used as the nanoparticles. Known albumins, which long-term circulate in blood and maintain aggregation in order to stably deliver drugs and are cancer-targeting, are preferably used for the nanoparticls. The albumin may be HSA or HSA fragments. The nanoparticles may have a diameter of 156.02± 65.76 nm or, preferably, a diameter that ranges from 100 to 300 nm.

The drugs to be internally filled in the nanoparticles may be unrestrictedly selected from those materials that can be loaded to known abumins and may be one or more that are selected from the group comprising docetaxel, cis-platin, camptothecin, PTX, tamoxifen, anasterozole, gleevec, 5-FU, floxuridine, leuprolide, flutamide, zoledronate, doxorubicin, vincristine, gemcitabine, streptozocin, carboplatin, topotecan, belotecan, irinotecan, vinorelbine, hydroxyurea, valrubicin, retinoic acid drugs, methotrexate, meclorethamine, chlorambucil, busulfan, doxifluridine, vinblastin, mitomycin, prednisone, testosterone, mitoxantron, aspirin, salicylates, ibuprofen, naproxen, fenoprofen, indomethacin, phenyltazone, cyclophosphamide, mechlorethamine, dexamethasone, prednisolone, celecoxib, valdecoxib, nimesulide, cortisone and corticosteroid.

The nanoparticles may be combined onto the microbubbles by means of the linkers or activated reactive groups on the microbubble surface. The reactive group may be thiol radicals or amine radicals while the linker may be a compound that includes the reactive group.

More specifically, the microbubbles and the nanoparticles may be amide-bonded to each other.

Such bond may be formed via amid bond between carboxyl radicals in the microbubbles and a plurality of amine radicals included in the albumin.

Another embodiment of the present invention relates to a method for preparing an ultrasound contrast agent including a phase that fabricates microbubbles and nanoparticles that contains drugs, respectively; and a phase that mixes the nanoparticles and microbubbles with water at a predetermined ratio so that the nanoparticles and the microbubbles react to each other.

FIG 2 illustrates a fabrication process of an ultrasound contrast agent according to the present invention. As illustrated in FIG 2, the microbubble fabrication process may comprise a phase that mixes phospholipid with an organic solvent to form thin lipid film; and a phase that hydrates the thin lipid film in water, injects gas, maintaining a high-pressure of the gas, in the thin lipid film and ultrasonically treats the thin lipid film.

Preferably, the phase to form thin lipid film may include a phase that mixes phospholipid, an NHS-containing phospholipid derivative and an emulsifier with an organic solvent.

For this purpose, the phase to form thin lipid film may mix an emulsifier, lipid and an NHS-containing phospholipid derivative at a molar ratio of 1 to 4: 7 to 9: 1 to 3.

The amphiphilic phospholipid compound aforementioned may be used as the phospholipid and NHS-containing phospholipid derivative.

In addition, lysolipid, stearic acid, polyethyleneglycol, polyoxyethylene fatty acid ester, polyoxyethylene fatty acid stearate, polyoxyethylene fatty alcohol, etc. may be used as the emulsifier.

The method to hydrate the thin lipid film and fill gas in the thin lipid film may employ a known method, for example, that puts and dissolves at 55 to 60°C a mixture of water, glycol and glycerin in a container that contains thin lipid film then ultrasonically treats the mixture by injecting gas at 200 kPa or combines an ultrasound treatment and a mechanical agitation. The drug-containing nanoparticle fabrication process comprises a phase that prepares a mixture by dissolving albumin in water then injecting drugs thereto and a phase that titrates the mixture so that the mixture has a pH that ranges from 7 to 10, preferably, 8.0 to 8.5 then instills alcohols, via the two phases of which the albumin forms self-aggregates.

The phase in which the nanoparticles react to the microbubbles bonds the nanoparticles on the microbubble surface by means of the linker- or reactive group-derived phospholipid. The linker or reactive group may be thiol radicals, amine radicals, biotin-avidin conjugates, etc. More specifically, the microbubbles and the nanoparticles may be amide-bonded to each other. Such bond may be formed via amid bond between carboxyl radicals in the microbubbles and a plurality of amine radicals included in the albumin. Further specifically, after the NHS on the microbubble surface is hydrolyzed in water, the carboxyl radicals that remain on the microbubble surface amide bond to the amine radicals on the nanoparticles.

The ratio at which the nanoparticles are mixed with the microbubbles may be 1: 0.5 to 2 in terms of the molar ratio of the reactive groups.

A preferred embodiment will be described in order to help understand the present invention, not to limit the present invention thereto.

### Embodiment 1

### Microbubble fabrication

DSPC as well as DSPE-PEG2000-NHS(1,2-dioleoyl-sn-glycero-3-phosphoethanolamine-n-[poly(ethyleneglycol)]₂₀₀₀-N-hydroxysuccinimide) both as lipid was mixed with POE40s(polyoxyethylene 40 stearate) as an emulsifier at a molar ratio of 8:1:1 and dissolved in chloroform. The chloroform was completely evaporated using a rotary evaporator to form thin lipid film.

In the next phase, distilled water, prophylene glycol and glycerin were mixed together at a proportion of 8:1:1 and the mixture was added to the thin lipid film. The lipid was dissolved with the temperature maintained at 55 to 60°C. Either SF6 or C3F8 in gaseous phase was injected to the mixture container and filled at 200 kPa and processed via both sonication and mechanical agitation, which fabricates the microbubbles.

### Nanoparticle(HSA-NPs) fabrication

40 mg of HSA was dissolved in 1 mL of distilled water in a vial and 100 to 200 µL of doxorubicin hydrochloride(5mg/mL) was added to the HSA-dissolved vial. The mixture was titrated with KOH or NaOH so that the pH of the mixture was 8.0 to 8.5 then titrated again by instilling 3 to 6 mL of ethanol with a rate of 1 mL/min. Should the HSA be aggregated for the mixture to be cloudy, 8%-glutaraldehyde was added as a linker to initiate reaction. In the next phase, the ethanol was completely evaporated and remaining mixture was centrifuged at 12,000rpm, 4°C for 10 minutes. Supernatant was removed with sedimented pellets kept. Unparticlized HSA and doxorubicin was removed and the remaining was washed with distilled water. The remaining was centrifuged at 3,000 rpm, 4°C for 5 minutes to remove particulates on the order of a micrometer then doxorubicin-loaded, the diameter of which ranged from 100 to 200 nm, HSA-NPs were extracted.

### Bond between the microbubbles and nanoparticles

The HSA-NPs and the microbubbles at a molar ratio of 1:0.5 to 2 were mixed at room temperature for 2 hours so that the nanoparticles were amide-bonded to the microbubbles. Unbonded HSA-NPs were removed in a centrifuge.

For information purpose, a higher bonding efficiency may be obtained by preliminarily adding and making react to each other an enough quantity of EDC and NHS in order to precautionarily compensate for the NHS loss incurred during the microbubble fabrication then by washing out residual EDC and NHS.

### Comparative Example 1

In Comparative Example 1, the microbubbles fabricated in Embodiment 1 were used, wherein the nanoparticles were not bonded to the microbubbles.

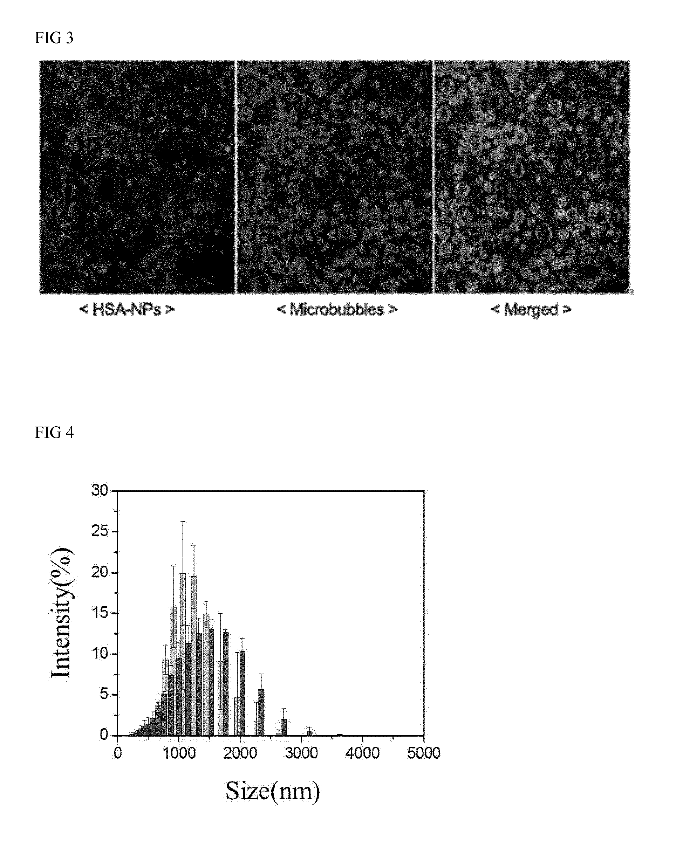

FIG 3 illustrates confocal fluorescence microscopic images of microbubbles, HSA-NPs(human serum albumin nanoparticles) that contain drugs, and an ultrasound contrast agent that combines the microbubbles and HSA-NPs, respectively, according to the present invention while FIG 4 illustrates size distribution of an ultrasound contrast agent combined with HSA-NPs according to the present invention.

More specifically, FIG 3 illustrates the test results to ascertain, by means of a fluorescence dye, whether or not the HSA-NPs had been bonded. For fluorescence dyeing, FITC and DiIC18 were used for green and red, respectively. In other words, FITC-containing bovine serum albumin was added by 10 to 20% in fabricating HSA-NPs to dye the HSA-NPs green while liphophilic DiIC18 was loaded between the microbubble bilayers in fabricating microbubbles to label the microbubbles. It is ascertained that the HSA-NPs are well attached to the microbubble surface with reference to the photo images merged in FIG 3.

FIG 4 illustrates dynamic light scattering results to plot the size distribution of HSA-NPs-unbonded(gray) and HSA-NPs-bonded(red) microbubbles, respectively. It is ascertained that HSA-NPs bonding slightly shifts the size distribution to the right and the size is mainly around 1,000 to 2,000 nm.

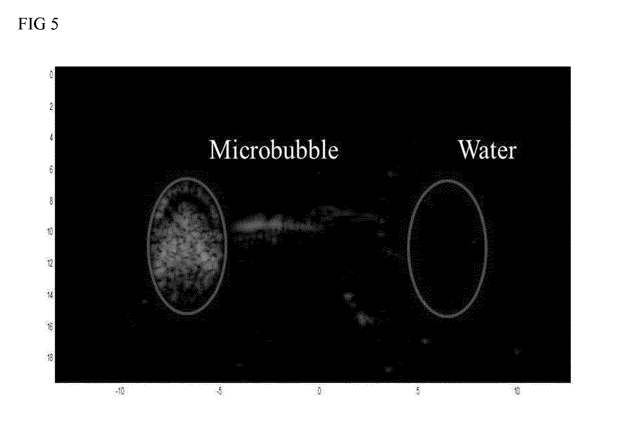

FIG 5 illustrates an ultrasound image of Embodiment 1 of the present invention and water as a control, both in an agarose phantom, taken by a commercial ultrasound diagnosis device in harmonic mode. Very weak ultrasound waves the intensity or mechanical index, MI, of which is 0.16 were used and a transducer that emits ultrasound waves of 5 to 12 MHz was used. As illustrated in FIG 5, the ultrasound contrast effect was superiorly expressed in Comparative Example 1 for HSA-NPs-bonded microbubbles to water the ultrasound contrast effect of which is almost invisible.

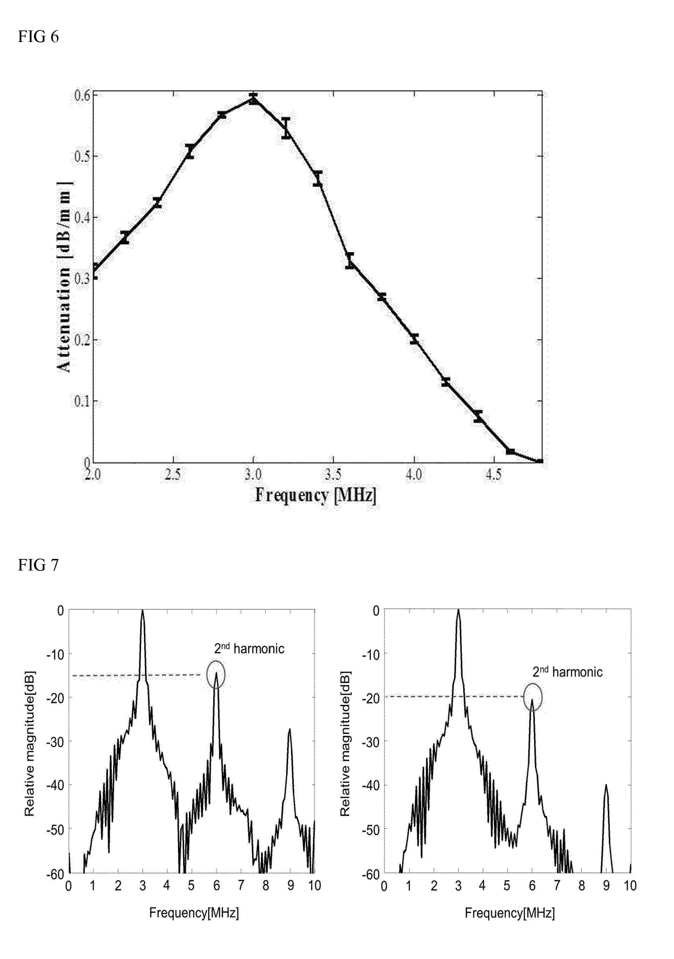

FIG 6 illustrates the harmonic elements selectively collected among the ultrasound waves, which reflected from or transmitted an ultrasound contrast agent according to the present invention, obtained by a hydrophone. A single transducer that can emit only a single wavelength was used in this experiment, wherein the transducer emitted, at a frequency of 2 MHz to 5 MHz at an interval of 0.2 MHz, ultrasound waves with an identical intensity(MI:0.1, cycle:20). The ultrasound waves emitted by the transducer reflected from or transmitted the ultrasound contrast agents, contained in an agarose phantom, that were bonded to the HSA-NPs thus fabricated. The ultrasound waves were received by a hydrophone and, among the entire ultrasound elements, only harmonic elements were detected and analyzed. As illustrated in FIG 6, the HSA-NPs-boned microbubbles transmitted the largest harmonic signals at 3 MHz, which indicates that the ultrasound frequency that maximizes the activity of the HSA-NPs-bonded microbubbles of Embodiment 1 is 3 MHz. 3MHz is a reasonable frequency to image human internal organs.

FIG 7 illustrates peaks the HSA-NPs-combined microbubbles of Embodiment 1 (left) and water (right), respectively, emit after being emitted by ultrasound waves at 3 MHz. The second peaks circled in red were obtained from the harmonic elements of the entire ultrasound elements. As illustrated in FIG 7, the peak intensity received from the ultrasound contrast agent of Embodiment 1 is higher by about 6 dB than the peak received from water, which indicates that, because Embodiment 1 emits a far higher intensity of ultrasound signals, Embodiment 1 acts as a contrast agent taking effects to a sufficient extent.

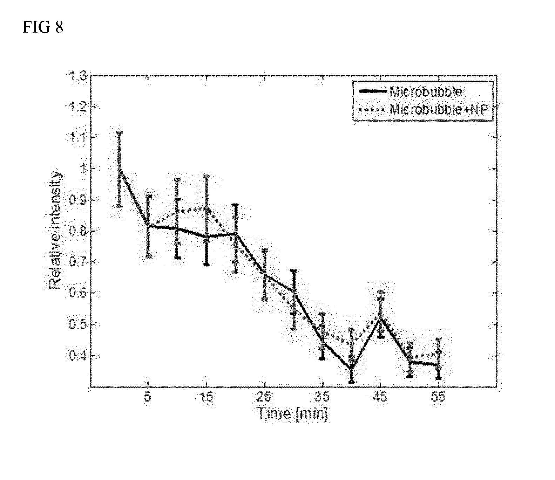

FIG 8 illustrates long-term stability, against ultrasound waves, of an ultrasound contrast agent of Comparative Example 1 that provides HSA-NPs-unbonded-microbubbles and Embodiment 1, respectively, according to the present invention. The stability was evaluated, under an identical condition to that of the ultrasound imaging experiment as shown in FIG 5, by shotting images at 20 fps and quantifying their intensity by means of Matlab. As illustrated in FIG 8, the intensity decreases over time because the microbubbles burst by ultrasound waves thus emitted. Embodiment 1 and Comparative Example 1 show a similar pattern of intensity decrease to each other, which indicates that nanoparticle bonding to Embodiment 1 impaired neither the stability nor imaging effect of the microbubbles.

### Antitumor effect at the cellular level

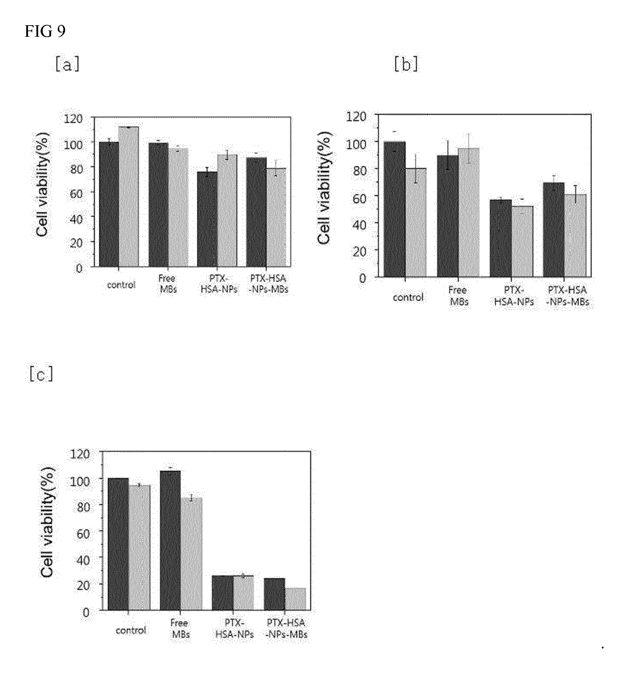

Antitumor effects were evaluated at the cellular level on MCF-7 using HSA-NPs that contained PTX, the antitumor agent. FIG 9 shows the antitumor effects after 6 (a), 24 (b) and 72 hours (c), respectively. Drug penetration effects into cells were also evaluated by emitting ultrasound waves at 3 MHz, the optimal frequency found in this experiment. 4 groups in total were evaluated comprising control, Comparative Example 1 that provides HSA-NPs-unbonded microbubbles, Reference Example 1 that provides PTX-containing HSA-NPs and Embodiment 1 that provides PTX-containing HSA-NPs-bonded microbubbles while the groups were divided depending on whether or not they were irradiated by ultrasound waves. Antitumor effects were analyzed by the MTT assay which is widely used to detect a survival rate of cells, the results of which are shown below and in FIG 9.
(6h: control; 100.00± 2.41%, 111.76± 0.50% (US+), free-microbubble; 99.25± 1.82%, 94.38± 2.14% (US+), PTX loaded HSA-NPs; 75.90± 3.55%, 89.68± 3.64% (US+), PTX loaded HSA-NPs conjugated microbubble;87.35± 3.85%, 78.90± 5.93% (US+)
24h control; 100± 7.28%, 80.01± 10.58% (US+), free-microbubble; 89.97± 10.68, 95.23± 9.59 (US+), PTX loaded HSA-NPs; 56.86± 2.26%, 52.39± 5.13% (US+), PTX loaded HSA-NPs conjugated microbubble; 69.72± 5.26%, 61.15± 6.54% (US+),
72h: control; 100.00± 0.08%, 94.67± 1.43% (US+), free-microbubble; 105.22± 2.72%, 85.28± 2.68% (US+), PTX loaded HSA-NPs; 26.37± 0.08%, 26.32± 1.25% (US+), PTX loaded HSA-NPs conjugated microbubble; 24.26± 0.05%, 16.53± 0.07% (US+)

With reference to FIG 9 and the results aforementioned, the survival rate of cancer cells went down below 30% after about 72 hours, which means sufficient antitumor effects. Meanwhile, Reference Example 1 and Embodiment 1 show similar antitumor effects to each other, which is considered because a frequency of 3 MHz, as optimized for imaging, was used. In other words, it is considered that the ultrasound contrast agent did not burst, not forming pores on cell walls, because a lower MI of 0.1 was used in order to sustain the imaging duration by the ultrasound contrast agent. For information purpose, it is expected that, if the microbubbles burst in Embodiment 1 by emitting ultrasound waves with a lower frequency and a higher intensity, pores may form on cell walls, which increases antitumor agent penetrating effects into cell walls so that the antitumor effects of Embodiment 1 are higher than those of Reference Example 1.

### Animal study

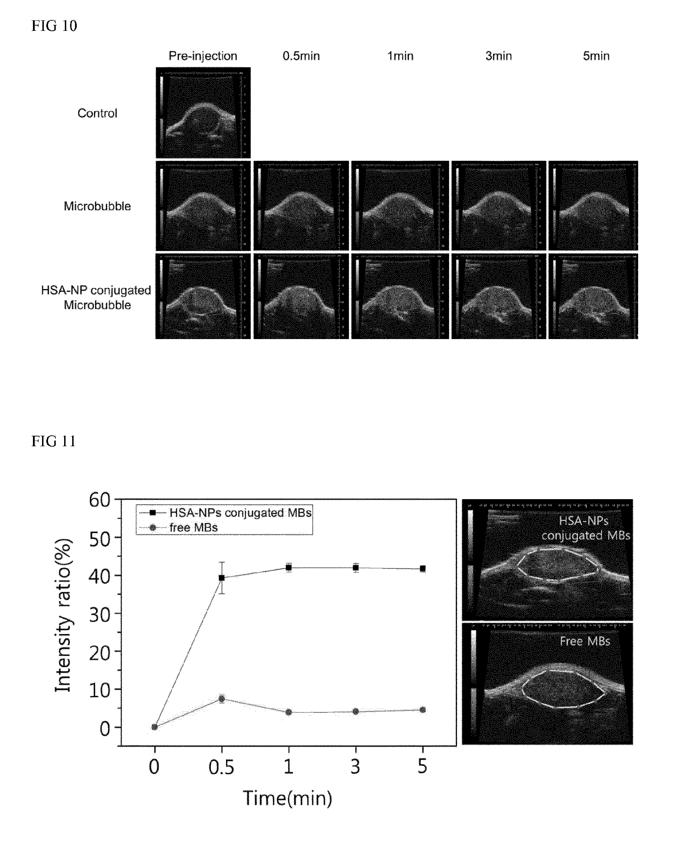

The ultrasound imaging effects of Embodiment 1 and Comparative Example 1 were verified via an animal model employing MCF-7 transplanted Balb/C nude mice. Each material was intravenously injected and ultrasound images pertinent to it were obtained by time. Visualsonics that is typically used in animal studies was uses at 40 MHz as an ultrasound imaging device. With the image obtained before the injection at baseline, the area where the intensity increased after injecting the material by ultrasound contrast agent was mapped in green to promote visibility as shown in FIG 10.

It is ascertained that Embodiment 1 reveals far superior imaging effects to that of Comparative Example as shown in FIG 10. Or, the imaging effect sustains over time in Embodiment 1 whereas Comparative Example 1 loses the effect immediately.

FIG 11 a Matlab plot that illustrates the intensity for quantitative comparison, in a tumor, of the imaging effects illustrated in FIG 10. With reference to FIG 11, it is ascertained that Embodiment 1 shows a far higher intensity than Comparative Example 1 in a tumor.

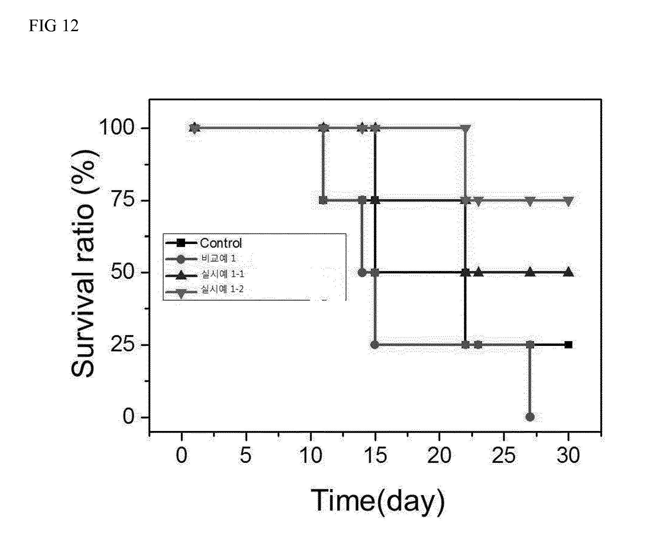

FIG 12 illustrates the survival ratio of the animal model employing MCF-7-transplanted Balb/C nude mice the results of which were obtained at the time the model showed a symptom of 0.5 mm.

4 groups in total were evaluated comprising control that was injected with PBS, Comparative Example 1 that provides HSA-NPs-unbonded microbubbles, Embodiment 1-1 that did not irradiate ultrasound waves onto Embodiment 1 that provides PTX-containing HSA-NPs-bonded microbubbles, Embodiment 1-2 that did irradiate onto Embodiment, with N = 4 for each group. In order to irradiate ultrasound waves, microbubbles burst for 10 minutes in the microbubble destruction mode of Visualsonics as used in the ultrasound imaging experiment.

This experiment was implemented once every fourth day, wherein the experiment animals were observed every day to check out their survival.

In control and Comparative Example 1, the animals commenced to die one by one after about 10 days, and most of the animals died after 23 days leaving only one surviving in control and Comparative Example, respectively. In Embodiment 1-1, one died after 14 days and another one died after 23 days while, in Embodiment 1-2, only one died after 20 days leaving three surviving at the end of the experiment.

Embodiment 1-1 shows antitumor effects some way short of that of Embodiment 1-2 because the PTX-HSA-NPs cannot penetrate their HSA-NPs into tumor tissue but elute their antitumor agents in blood vessels, which impairs drug delivery efficacy into cancer cells.

On the contrary, it is considered that Embodiment 1-2 showed effective antitumor effects because Embodiment 1-2 that provides PTX-HSA-NPs-MB(US+) induced the HSA-NPs to effectively penetrate into blood vessel tissue and be delivered to cancer tissue by bursting its microbubbles in the proximity of cancer tissue by means of ultrasound waves. In addition, the HSA-NPs are transported across the interior or a cell by gp60 receptors to be delivered into cancer cells, which also increased the antitumor effects. Furthermore, it is considered that the delivery effect also increased by forming pores on the cell walls of cancer tissue because the microbubbles experienced cavitation by means of the ultrasound waves irradiated from their exterior.

Any predictable modification or change of the present invention must be included in the scope of the present invention and the scope of the protection of the present invention should be clarified based on the claims of the present invention.

### Reference Numerals

10: Microbubble
20: Nanoparticle
21: Drug
30: Linker

## Claims

1. An ultrasound contrast agent comprising:
microbubbles internally filled with gas; and
nanoparticles that contain drug and are bonded onto the surfaces of the microbubbles.

2. The ultrasound contrast agent of claim 1, wherein the nanoparticles internally contain drugs and form self-aggregates such as albumin.

3. The ultrasound contrast agent of claim 1, wherein the nanoparticles have a diameter that ranges from 100 to 300 nm.

4. The ultrasound contrast agent of claim 1, wherein the albumin is Human Serum Albumin (HSA) or HSA fragments.

5. The ultrasound contrast agent of claim 1, wherein the drugs are one or more that are selected from the group comprising docetaxel, cis-platin, camptothecin, PTX, tamoxifen, anasterozole, gleevec, 5-FU, floxuridine, leuprolide, flutamide, zoledronate, doxorubicin, vincristine, gemcitabine, streptozocin, carboplatin, topotecan, belotecan, irinotecan, vinorelbine, hydroxyurea, valrubicin, retinoic acid drugs, methotrexate, meclorethamine, chlorambucil, busulfan, doxifluridine, vinblastin, mitomycin, prednisone, testosterone, mitoxantron, aspirin, salicylates, ibuprofen, naproxen, fenoprofen, indomethacin, phenyltazone, cyclophosphamide, mechlorethamine, dexamethasone, prednisolone, celecoxib, valdecoxib, nimesulide, cortisone and corticosteroid.

6. The ultrasound contrast agent of claim 1, wherein the microbubbles are microspheres filled with gas, liposomes filled with gas or gas-forming emulsions.

7. The ultrasound contrast agent of claim 1, wherein the nanoparticles are bouned to the microbubbles by means of thiol functional radicals (disulfide bonding), amine functional radicals (amide bonding) and biotin-avidin conjugates.

8. The ultrasound contrast agent of claim 1, wherein the microbubbles and the nanoparticles are bound via an amide-bond to each other.

9. The ultrasound contrast agent of claim 1, wherein the microbubbles have a diameter that ranges from 0.1 to 10 µm.

10. A method for preparing an ultrasound contrast agent including:
a phase that fabricates microbubbles and nanoparticles that contains drugs, respectively; and
a phase that mixes the nanoparticles and microbubbles with water at a predetermined ratio so that the nanoparticles and the microbubbles are bounded to each other.

11. The method for preparing an ultrasound contrast agent of claim 10,
wherein the phase that fabricates microbubbles comprises:
a phase that mixes lipid, an NHS-containing lipid derivative and an emulsifier with an organic solvent to form thin lipid film; and
a phase that hydrates the thin lipid film in water, injects gas, maintaining a high-pressure of the gas, in the thin lipid film and ultrasonically treats the thin lipid film.

12. The method for preparing an ultrasound contrast agent of claim 11, wherein the phase to form thin lipid film mixes an emulsifier, lipid and an NHS-containing phospholipid derivative at a molar ratio of 1 to 4: 7 to 9: 1 to 3.

13. The method for preparing an ultrasound contrast agent of claim 10,
wherein the phase that fabricates drug-containing nanoparticles comprises:
a phase that prepares a mixture by dissolving albumin in water then injecting drugs thereto; and
a phase that titrates the mixture so that the mixture has a pH that ranges from 7 to 9, via the two phases of which the albumin forms self-aggregates

14. The method for preparing an ultrasound contrast agent of claim 10, wherein, after the NHS on the microbubble surface is hydrolyzed in water, the carboxyl radicals that remain on the microbubble surface amide bond to the amine radicals on the nanoparticles.

15. The method for preparing an ultrasound contrast agent of claim 10, wherein the ratio at which the nanoparticles are mixed with the microbubbles is 1: 0.5 to 2 in terms of the molar ratio of the reactive groups.
